# EUROPEAN PATENT APPLICATION

(11) **EP 4 079 263 A1**
(43) Date of publication of application: **26.10.2022**
(21) Application number: 20906930.1
(22) Date of filing: 22.12.2020
(51) Int. Cl.: A61F 2/36, A61F 2/32

(54) **WING-SHAPED STEMLESS HIP PROSTHESIS**

(30) Priority: 23.12.2019 CN 201911342256
(71) Applicant: Zhu, Hongwen, Tianjin 300071 (CN)
(72) Inventor: HUANG, Guofu, Tianjin 300071 (CN); DONG, Ronghua, Tianjin 300071 (CN); ZHU, Tianmou, Tianjin 300071 (CN); ZHU, Hongwen, Tianjin 300071 (CN)
(74) Representative: Sun, Yiming
(86) International application number: PCT/CN2020/138378
(87) International publication number: WO 2021/129622

(57) **Abstract**

The present invention discloses a wing-shaped stemless hip joint prosthesis, comprising a wing-shaped cup body which can be tightly covered on the femoral neck and the intertrochanter to fix an artificial spherical end and the femoral tissue into a whole, wherein the wing-shaped cup body integrally in an inverted cup form is inversely buckled outside the residual end of the femoral neck and is reinforced through a center lock pin and a tail pin; the wing-shaped cup body is provided with a lesser trochanter protecting wing buckled outside the lesser trochanter and a cup wing covered outside the intertrochanter line to acquire the additional supports and to be more stable accordingly; and the wing-shaped cup body is further provided with a large opening or hole so as to contain the nourishing blood vessels of the femur, accordingly decreasing the damage rate of the nourishing blood vessels and improving the postoperative recovery effect.

## Description

### Technical Field

The present invention generally relates to a medical artificial joint prosthesis, and more particularly relates to a buffered/non-buffered wing-shaped stemless hip joint prosthesis.

### Background Art

The human hip joint is a spherical joint formed between the thigh bone (femur) and the pelvis, including a spherical femoral head and a bowl-like acetabulum. The outer surface of the spherical femoral head and the inner surface of the bowl-like acetabulum are covered with smooth cartilage, and the synovial membrane can secrete mucus, both of which allow friction to be minimized. However, when the hip joint develops lesions, the cartilage is no longer smooth and the synovial sac shrinks due to inflammation, so the hip joint can no longer function as usual. In case that the symptoms such as pain, stiffness or deformation, and even inconvenient movement caused by degeneration, lesion, or trauma of the hip joint cannot be relieved by drugs or other treatments, the replacement of an artificial hip joint generally will be performed by surgery for the patient, so that the patient can restore normal activities.

The artificial hip joint prosthesis imitates the structure of the human hip joint, including a prosthetic metal cup acting as the acetabulum and a spherical end acting as the femoral head, wherein the spherical end can be fixed on the human femur via a femoral stem provided thereon.

In recent decades, most of the spherical end replacement surgeries performed in China utilize stemmed hip joint prosthesis, which is provided with a bolt at the lower end of the femoral stem to be inserted into the femoral medullary cavity so as to fix the femoral stem on the human femur.

For example, the hip joint prostheses have been disclosed in the patent documents such as the Patent No. 86201016 with the title of "ARTIFICIAL HIP BONE WITH BONE SEPARATING MOMENT FORCE" and the Patent No. 90225988.1 with the title of "LOCK LOOP TYPE SERIES OF ARTIFICIAL HIP JOINTS". However, such stemmed hip joint prosthesis has many disadvantages as described below:
(1) a large area of the physiological structure has to be removed from the femur when a stemmed hip joint prosthesis is implanted, which causes large intraoperative bleeding and later also has a influence on blood supply and physiological metabolism of the femoral medullary cavity;
(2) after used for a period of time, the tightness between the femoral stem of the stemmed hip joint prosthesis and the femur where the femoral stem is located gradually decreases, so that the connection between them becomes loose; and under the action of body pressure, the femoral stem and the bolt thereon sink further, resulting in different lengths of the patient's two legs, which will further affect the normal physiological function of the hip joint;
(3) the stemmed hip joint prosthesis is limited by its service life, since in fact it is difficult to be used more than 20 years and also difficult to be replaced in the future, and even in some cases the breakage of the stem may occur; and the stemmed hip joint prosthesis is also limited by the age of the patient which is usually required to be over 45 years old.

In order to overcome the above shortcomings, the inventors have made a great effort for more than 30 years and gradually developed a stemless hip joint prosthesis. Part of the studies have been disclosed in the patent documents publicated successively, such as the patent application CN 98200233.5 with the title of "STEMLESS ANATOMICAL ARTIFICIAL HIP JOINT", the patent application No. 00244070.9 with the title of "SELF-LOCKING STEMLESS ARTIFICIAL HIP JOINT", the patent application No. 200520027479.4 with the title of "ASSEMBLED BUFFER STEMLESS HIP JOINT", and the patent application No.201230597170 with the title of "ARTIFICIAL STEMLESS HIP JOINT SHELL". The stemless hip joint prostheses disclosed in above documents have overcome some of the shortcomings with the stem hip joint prostheses, but some of the problems still cannot be solved completely. Moreover, with the gradual progress of society, increasingly higher cure standards, cure limitation and life quality for the patients are required by the doctors, and correspondingly, more problems will need to be further solved.

In view of the above reasons, the inventors made a deep research to the prior stemless prostheses, and have designed a novel wing-shaped stemless hip joint prosthesis which is capable to overcome the above problems.

### Summary

In order to solve the problems as described above, the inventors have made a great effort to research and developed a wing-shaped stemless hip joint prosthesis, comprising:
a wing-shaped cup body covered outside the residual end of the femoral neck, via which an artificial spherical end and the femoral tissues are fixed together as a whole; and
a buffer device arranged between the artificial spherical end and the wing-shaped cup body, via which the application effect of the joint prosthesis can be improved;
wherein the wing-shaped cup body integrally in an inverted cup form is inversely buckled outside the residual end of the femoral neck and is reinforced through a center lock pin and a tail pin; the wing-shaped cup body is provided with a lesser trochanter protecting wing buckled outside the lesser trochanter and a cup wing covered outside the intertrochanter line to acquire the additional supports and to be more stable accordingly; and the wing-shaped cup body is further provided with a large opening or hole so as to contain the nourishing blood vessels of the femur, accordingly decreasing the damage rate of the nourishing blood vessels and improving the postoperative recovery effect.

Specifically, one object of the present disclosure is to provide a wing-shaped stemless hip joint prosthesis, comprising a wing-shaped cup body 6 that can be tightly covered outside the femoral neck and the intertrochanter 31, wherein the front of the wing-shaped cup body 6 is opened, accordingly exposing the residual end of the femoral neck therein; a lesser trochanter protecting wing 10 is extended obliquely downward from the front of the wing-shaped cup body 6 and can be tightly buckled outside the lesser trochanter 32; and a cup wing 7 is extended obliquely downward from the side of the wing-shaped cup body 6 and can be fixed on the intertrochanter line 33.

The present invention has the beneficial effects as following:
(1) the wing-shaped stemless hip joint prosthesis provided according to the present invention is fixed through the wing-shaped cup body inversely buckled outside the residual end of the femoral neck, thereby causing less damage to native femoral tissue and reducing bleeding during surgery;
(2) the wing-shaped stemless hip joint prosthesis provided according to the present invention is provided with a lesser trochanter protecting wing and a cup wing, which afford the additional positions bearing force for the wing-shaped cup body to allow a more stable connection between the wing-shaped cup body and the femur;
(3) the wing-shaped stemless hip joint prosthesis provided according to the present invention is provided with a large window or hole, thereby retaining more blood supply channels for the femur;
(4) the wing-shaped stemless hip joint prosthesis provided according to the present invention is provided with a buffer device inside the artificial spherical end, thereby further improving the buffering and the shock absorption effects; and
(5) during implantation, the femoral tissue is subjected to less damage from the wing-shaped cup body of the wing-shaped stemless hip joint prosthesis provided according to the present invention, which is only comparable to the damage level during preliminary steps of a stem joint replacement surgery, so that the stemless hip joint prosthesis can be easily replaced when it reaches the service life.

### Description of Figures

FIG. 1 illustrates a schematic structural diagram of the wing-shaped stemless hip joint prosthesis according to a preferred embodiment of the present invention arranged on the femur at one angle;
FIG. 2 illustrates a schematic structural diagram of the wing-shaped stemless hip joint prosthesis according to a preferred embodiment of the present invention arranged on the femur at another angle;
FIG. 3 illustrates a schematic structural diagram of the wing-shaped stemless hip joint prosthesis according to a preferred embodiment of the present invention arranged on the femur at still another angle;
FIG. 4 illustrates a schematic diagram of the overall structure of the wing-shaped stemless hip joint prosthesis according to ae preferred embodiment of the present invention;
FIG. 5 illustrates a schematic structural diagram of the neck rod completely extending into the neck rod hole in the wing-shaped stemless hip joint prosthesis according to a preferred embodiment of the present invention;
FIG. 6 illustrates a schematic structural diagram of the neck rod ejected outward by the buffer device to the maximum stroke in the wing-shaped stemless hip joint prosthesis according to a preferred embodiment of the present invention;
FIG. 7 illustrates a schematic structural diagram of a limit cavity and a limit protrusion in the wing-shaped stemless hip joint prosthesis according to a preferred embodiment of the present invention.

### Description of the Reference Signs:

1- artificial spherical end
11- neck rod hole
12- buffer device accommodating cavity
13- limit slot
2- buffer device
31- intertrochanter
32- lesser trochanter
33- intertrochanter line
4- neck rod
41- limit protrusion
42- limit cavity
43- compressed spring
5- main fixed table
51- neck tapered hole section
52- pin tapered hole section
6- wing-shaped cup body
61- tail wing
62- auxiliary fixed table
63- tapered hole
64- large window
7- cup wing
71- cup wing through hole
8- tail pin
9- center lock pin
10- lesser trochanter protecting wing

### Embodiments

The present invention will be further described in detail below through the accompanying drawings and embodiments. Through these descriptions, the characteristics and advantages of the present invention will become clearer.

The term "exemplary" used herein means "serving as an example, embodiment, or illustration". Any embodiment described herein as "exemplary" does not need to be construed as being superior or better than other embodiments. Although various aspects of the embodiments are shown in the drawings, unless otherwise stated, the drawings are not necessarily drawn to scale.

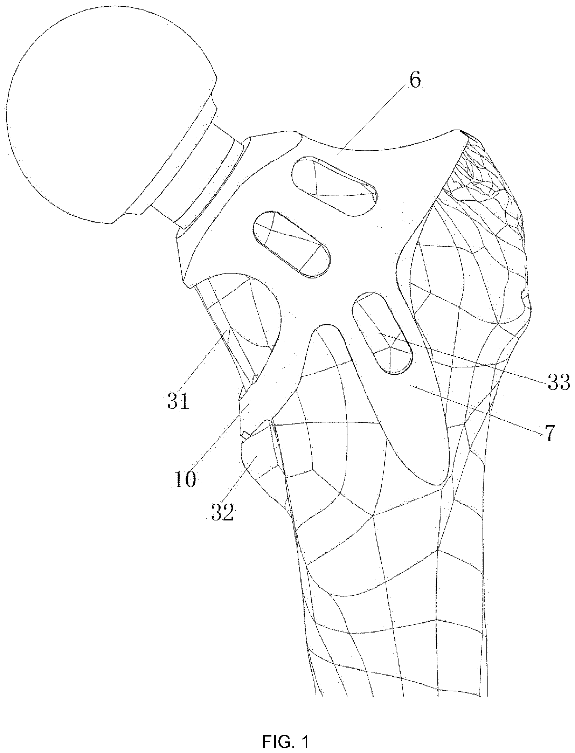

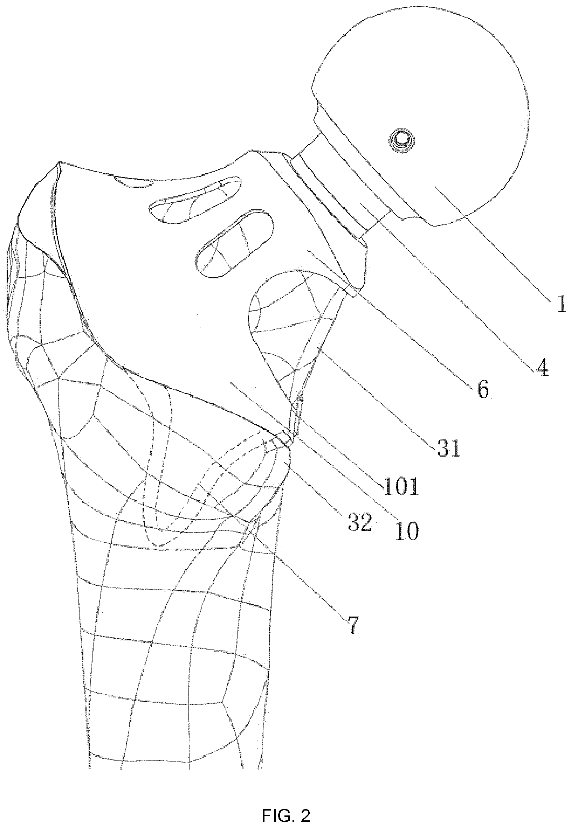

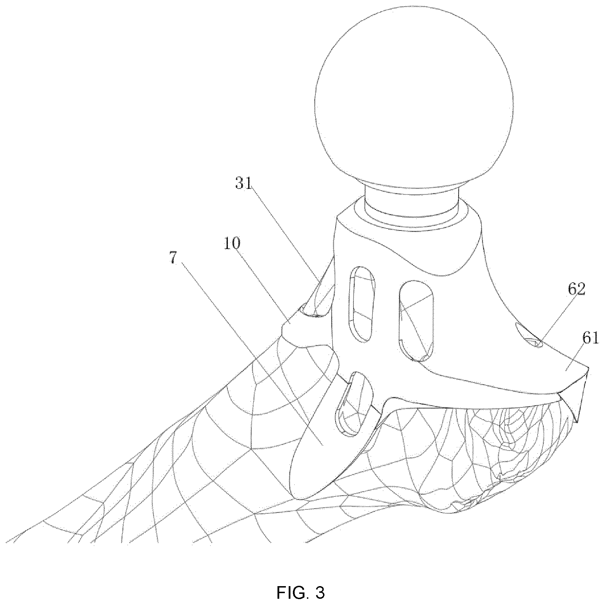

As shown in FIGs. 1-4, the wing-shaped stemless hip joint prosthesis according to the present invention comprises a wing-shaped cup body 6 that can be tightly covered outside the femoral neck and the intertrochanter 31. The wing-shaped cup body 6 has an inverted cup structure with an opening facing obliquely downward, so that the residual end of the femoral neck and the inner wall of the wing-shaped cup body 6 can be tightly attached to each other, accordingly achieving more stable installation of the wing-shaped cup body 6.

Before the wing-shaped cup body 6 described herein is installed, only the femoral head of the human femur needs to be removed, and most of the femoral neck can be reserved, and the greater trochanter and the lesser trochanter can be also substantially reserved. According to various anatomical structures of different people, a part of the cortical bone can be removed adaptively by thinning to facilitate the installation of the wing-shaped cup body 6. During the

Installation of the wing-shaped cup body 6, it is desired to ensure the installation strength and stability, meanwhile the bone tissue is subjected to less damaged as possible, accordingly protecting the blood supply channels and trabecular bone of the femur to maintain its physiological function.

The reserved part of the femoral neck is known as the residual end of the femoral neck. Since the installation of the wing-shaped cup body 6 described herein generates less damage to the bone tissues of the femoral neck and the intertrochanter, the femoral neck can be reserved more, and the intertrochanter can be approximately reserved completely, and even the tissues such as the ascending carotid artery among the tuberosities can also be reserved.

Although the damage to the femur caused by the existing stemless hip joint has been reduced, the physiological structure of the femur, especially the trabecular bone, still can be destroyed to some extent, which leads to the degraded hematopoietic function of the patient after surgery. Additionally, the problem of biocompatibility between the implanted prosthesis and the human body is still difficult to be solved completely.

Compared to using the existing stemless hip joint, the application of the wing-shaped cup body 6 according to the present invention can allow the femoral neck to be reserved at least 10-20% more, and moreover, can further allow the force applied on the femur to be dispersed, accordingly reducing the shear force on the femoral tissues. Additionally, the space for the growing of the nourishing blood vessels has been preserved on the wing-shaped cup body 6, and therefore the residual femur can grow normally.

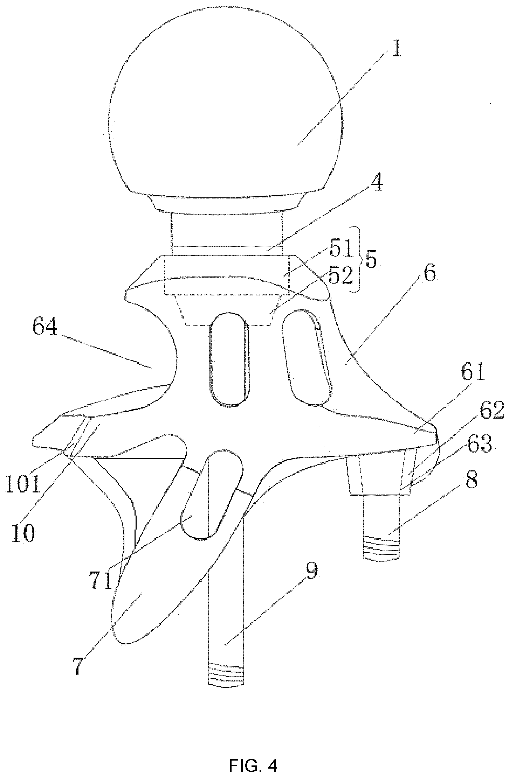

The front of the wing-shaped cup body 6 is opened, accordingly exposing the residual end of the femoral neck and the intertrochanter therein. The opening at the front is also known as a large window structure as shown by the mark 64 in FIG. 4, which can allow the nourishing blood vessels outside the femoral neck to be reserved and can also prevent the trabecular bone in the femoral neck from being damaged.

Specifically, after the installation of the wing-shaped cup body 6 is completed, the nourishing blood vessels at the large window, especially the position where the nourishing blood vessels enter and exit the femur, will not received the shear force from the wing-shaped cup body 6, and the nourishing blood vessels and the trabecular bone can maintain the normal physiological function and physiological support, so that the trabecular bone is not easily suffered from the disuse absorption of the femoral tissue too early, which can greatly reduce the incidence of postoperative necrosis of the femoral neck residual end.

As shown in FIG. 4, the left in the figure is the front as described herein, and the right in the figure is the rear as described herein.

The lesser trochanter protecting wing 10 is extended obliquely downward from the front of the wing-shaped cup body 6, and can be buckled outside the lesser trochanter 32, thereby providing a new limit fixing force for the joint prosthesis and accordingly increasing its firmness and stability. Since the lesser trochanter protecting wing 10 and the front of the wing-shaped cup body 6 are both located around the opening at the front, the large window structure can be formed.

Preferably, the lesser trochanter protecting wing 10 is closely attached to the lesser trochanter 32 and tightly buckled outside. More preferably, the lesser trochanter protecting wing 10 has elasticity, so that it can be deformed outwards when buckled outside the lesser trochanter 32 and therefore can be attached to the lesser trochanter 32 more closely under elastic force.

As shown in FIG. 1 and FIG. 4, when the wing-shaped cup body 6 is installed on the femur, the angle between the lesser trochanter protecting wing 10 and the axis of the neck rod 4 is in a range from 110° to 115°, preferably 112° to 113°. The inventors found that when the angle is 112° to 113°, the lesser trochanter protecting wing 10 can be stably connected to the less trochanter and is not tend to slip off. Moreover, the lesser trochanter protecting wing 10 can afford the wing-shaped cup body 6 sufficiently strong support to ensure the cup body 6 to be stably fixed on the femur.

According to the present invention, the lesser trochanter can be retained, and the lesser trochanter protecting wing 10 described herein extends along the top of the lesser trochanter and is embedded at the periphery of the top and inner side of the lesser trochanter. Since the protecting wing 10 covers the junction area between the less trochanter and the main part of the femur, it can further enhance the connection strength between the wing-shaped cup body 6 and the femur as an additional support.

The cup wing 7 is constructed to extend obliquely downwards from the side of the wing-shaped cup body 6. One or two cup wing(s) 7 can be arranged, and preferably two cup wings 7 are arranged each independently on two sides of the wing-shaped cup body 6, as shown in FIG. 1, FIG. 3 and FIG. 4. In this case, one cup wing 7 can extend obliquely downward to be embedded in the cancellous bone of the intertrochanteric line 33, and can be buckle fixed with the intertrochanteric line on the femur, accordingly providing additional force-bearing position for the fixation between wing-shaped cup body 6 and the femur and subsequently further improving the stability of the wing-shaped cup body; and the other cup wing 7 can be arranged on another side of the femur and closely attached to the outer surface of the femur, similarly providing additional force-bearing position for the fixation between wing-shaped cup body 6 and the femur.

The cup wing 7 is preferably designed to have a curvature, which is adapted to the physiological curvature of the human femur, especially the intertrochanteric line, so as to closely attach to the intertrochanteric line without excessively damage to the femoral structure.

The cup wing 7 is provided with a cup wing through hole 71, by which the femoral structure under the cup wing 7 can avoid to be damaged and accordingly the nourishing blood vessels outside the femur can maintain their physiological function.

In the process of implantation and fixation of the conventional stemmed joint prosthesis, the blood supply channels on the surface of the femoral tissue are subjected to great damage, and therefore, after implanted, the blood supply channels at the top area of the femur is difficult to be rebuilt. With regard to the existing stemless prosthesis, after implanted, some blood supply channels can be recovered at some interspaces, however, the compression around the femoral tissue surface still exists and the blood supply cannot be significantly satisfied. Additionally, the post-recovered blood supply channels have degraded performance compared to the native blood supply channels.

The wing-shaped cup body 6 according to the present invention has an asymmetrical curved edge at its opening, which allows the wing-shaped cup body 6 not to completely attach to the femoral surface with some space therebetween. Therefore, part of the blood supply channels on the femoral surface can be retained, new blood supply channels can be generated on the femoral surface at the space via compensatory hyperplasia, and additionally the compression around the end of the femur can be prevented from generating.

Further preferably, the part of the lesser trochanter protecting wing 10 close to the intertrochanter is protruded outward, and a concave channel 101 running through the lesser trochanter protecting wing 10 is formed in the interior thereof. A cavity is constructed between the concave channel 101 and the femur, so that the blood vessels and surrounding tissues attached to the outside of the femur, especially the ascending carotid artery, can be accommodated in the cavity.

The arrangement of the concave channel 101 can prevent the blood vessels and surrounding tissues from being damaged by shear force derived from the lesser trochanter protecting wing 10, to ensure body fluid to circulate smoothly inside and outside the wing-shaped cup body 6, avoid the necrosis of tissues and structures, and reduce the occurrence of complications.

Preferably, one or more concave channel(s) 101 can be arranged, and more preferably, two concave channels 101 can be arranged.

In another preferred embodiment, a curved notch is provided at the inside edge of the lesser trochanter protecting wing 10 and constructed to protrude outward, so that the ascending carotid artery of the femoral neck can pass through the curved notch and be arranged under the lesser trochanter protecting wing 10 to be protected. Further preferably, two sides constructing the curved notch on the lesser trochanter protecting wing 10 are bent toward the artificial spherical end, and are connected to each other away from the ascending carotid artery, so that the streaks caused by the concentrated stress between the lesser trochanter protecting wing 10 and the femur can avoid the area with abundant blood vessels, the impact of the installation of the wing-shaped cup body 6 on the blood supply channels of the femur can be reduced, and the postoperative recovery effect can be improved.

In a preferred embodiment, a cylindrical main fixed table 5 is provided inside the wing-shaped cup body 6 and is connected with the interior top surface of the cup body 6. The main fixed table 5 is used to fix the artificial spherical end 1 and the center lock pin 9 on the cup body 6, to realize the replacement of the femoral head prosthesis.

Preferably, a through hole can be provided inside the main fixed table 5, so that the center lock pin can be screwed in from the top of the wing-shaped cup body 6 and the neck rod on the artificial spherical end can be embedded and fixed in the cup body 6.

The front of the center lock pin 9 passes through the wing-shaped cup body 6, and then is screwed into the proximal end of the femoral tissue, that is, the position between the greater and lesser trochanter.

Preferably, the main fixed table 5 is protruded from the top of the wing-shaped cup body 6, that is, the top of the main fixed table is exposed outside the wing-shaped cup body 6. With such design of the structure, the connection between the main fixed table 5 and the wing-shaped cup body 6 can be strengthened, and the neck rod 4 can be easily aligned and fixed with the wing-shaped cup body 6.

The tail wing 61 is extended from the back of the wing-shaped cup body 6. The cylindrical auxiliary fixed table 62 is provided below the tail wing. A through hole is arranged inside the auxiliary fixed table and protruded from the top of the tail wing, so that the tail pin 8 can be screwed in from the top of the tail wing and fix the wing-shaped cup body 6.

The front of the tail pin 8 passes through the wing-shaped cup body 6, and then is screwed into the proximal end of the femoral tissue between the greater and lesser trochanter, more preferably near the greater trochanter.

The hip joint is an important buffer joint in the human body, which is desired to have the capacity of buffering. Few of the existing stemmed prosthesis takes into account the buffering effect, and some existing stemless prosthesis is related to buffer devices. For example, in the patent No. 200520027479.4 "ASSEMBLED BUFFER STEMLESS HIP JOINT", the impact force is relieved by providing a buffer shock absorber spring, but in practices, it is found that this solution has some shortcomings to be further improved. Specifically, the shock absorption of the human hip joint mainly depends on the cartilage near the femoral head, but in this solution, the buffer shock absorber spring is arranged close to the femoral prosthesis, which is different from the functional position on the human body, so that the connecting position between the femoral prosthesis and the femur is subjected directly to the action of the spring, and it is difficult to reduce the motion load of the human body, accordingly leading to the stress concentration and poor stability of the femoral prosthesis. Further, in this solution, the buffer shock absorber spring and its limit pin have to be installed in site, which is hard to operate and takes much time for the doctors, accordingly increasing the operation time.

In a preferred embodiment, the shape and size of the artificial spherical end 1 is designed according to the human femoral head. A spherical structure is arranged on the top of the artificial spherical end, and a through hole is arranged at the bottom of the artificial spherical end, so that one end of the neck rod can be inserted into the artificial spherical end, and the other end of the neck rod can be inserted into the main fixed table of the wing-shaped cup body 6.

The artificial spherical end herein can have or not have buffering function.

Since the artificial spherical end and the wing-shaped cup body are installed during the replacement surgery and totally implanted into the human body after installation without possibility to adjust again, there are quite high requirements for the connecting reliability between them, the service life, the tensile and compressive strength after connecting, and the speed and convenience of the connection.

Additionally, in the existing stemless hip joint prosthesis with the shock absorption function, the shock absorption structure is arranged inside the wing-shaped cup structure, which brings a series of problems.

Therefore, in a preferred embodiment of the present invention, the buffer device 2 is arranged inside the artificial spherical end 1. One end of the buffer device 2 is in contact with the inner wall of the artificial spherical end 1, and the other end is in contact with the neck rod 4, which is fixed on the wing-shaped cup body 6. The artificial spherical end 1 can move relative to the neck rod 4 to some extent. When a pressure is applied on the artificial spherical end 1, the artificial spherical end 1 can move downward to compress the buffer device 2. As the artificial spherical end 1 moves downward more and more, the buffer device 2 is gradually deformed, and the elastic force on it is gradually increasing. Finally the artificial spherical end 1 can be bounced back to the original position by the buffer device 2.

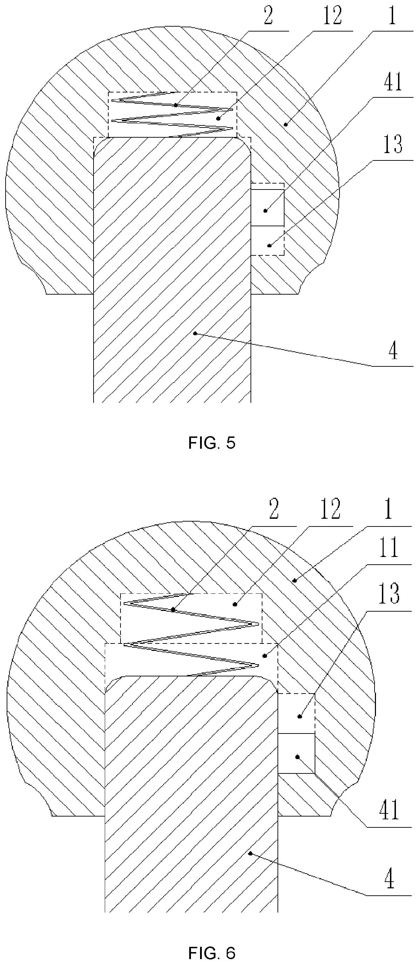

Further preferably, as shown in FIG. 5 and FIG. 6, the neck rod hole 11 for the neck rod inserted is provided in the artificial spherical end and its top is communicated with the buffer device accommodating cavity 12. The neck rod 4 can move back and forth in the neck rod hole 11. When the neck rod 4 is extended completely into the neck rod hole 11, the neck rod hole 11 can be basically filled. Since the diameter of the buffer device accommodating cavity 12 is smaller than that of the neck rod 4, the neck rod 4 cannot extend into the cavity, thereby leading to a limit in one direction for the neck rod 4. FIG. 5 shows the structural schematic diagram when the neck rod 4 is completely extended into the neck rod hole 11, and FIG. 6 shows the structural schematic diagram when the neck rod 4 is ejected to the maximum stroke by the buffer device 2. In these figures, the inner contour structure of the artificial spherical end 1 is indicated by the dashed line, and the outer contour structure of the artificial spherical end 1 and the neck rod 4 are indicated by the solid line.

Preferably, the cross-sectional dimension of the top shaft of the neck rod 4 is equal to or slightly smaller than that of the neck rod hole 11, so as to facilitate the neck rod 4 to move relative to the artificial spherical end 1.

Preferably, the limit slot 13 is arranged on the side wall of the accommodating cavity 12, and correspondingly the limit protrusion 41 extending to the side is provided on the neck rod 4. The limit protrusion 41 is embedded in the limit slot 13 and thereby can move together with the neck rod 4. With the cooperation between the limit protrusion 41 and the limit slot 13, the movement of the neck rod 4 can be limited in another direction, thereby ensuring that the neck rod 4 and the artificial spherical end 1 cannot be separated from each other.

During actual use, the artificial spherical end 1 is not only firmly connected and undetachable from the neck rod, but it is also necessary to ensure quick installation between the artificial spherical end 1 and the neck rod 4 with lower installation difficulty and time saving.

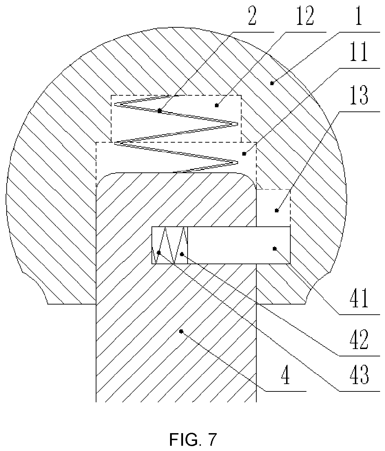

Preferably, as shown in FIG. 7, the limit cavity 42 is arranged laterally on the neck rod 4, and the limit protrusion 41 in the form of a long rod is substantially located in the limit cavity 42. The bottom of the limit cavity 42 is provided with a compressed spring 43. When it is needed to insert the neck rod 4 into the artificial spherical end 1, the limit protrusion 41 is pressed inwardly to be embedded totally in the limit cavity 42, and at the same time, the compressed spring 43 inside the cavity is pressed. When the limit protrusion 41 is close to the limit slot 13, the compressed spring 43 can push the end of the limit protrusion 41 out of the limit cavity 42, so that the limit protrusion 41 is inserted into the limit slot 13, thereby accomplishing the connection between the neck rod 4 and the artificial spherical end.

The buffer device 2 can be a spring, an elastic sheet or other elastic device made of biocompatible material, which is not particularly limited herein.

In order to not damage the medullary cavity, the existing stemless prosthesis does not have a long pin inserted into the medullary cavity, and thereby its fixation is not firm enough compared with the stemmed prosthesis. Additionally, the existing stemless prosthesis can not achieve sufficient force balance whether the self-locking screw is installed or not, so that after a period of time, the injury and deformation of the femur can not be avoided. With the increase of the use time, the movable space between the stemless prosthesis and the femur becomes larger and larger, which gradually affects the use effect and even leads to some other complications.

In a preferred embodiment, as shown in FIG. 4, the through holes on the main fixed table 5 include the neck tapered hole section 51 and the pin tapered hole section 52, both of which are tapered holes being gradually narrow from top to bottom.

The neck rod 4 is inserted into the neck tapered hole section 51 and fixed thereto. Preferably, the lower end of the neck rod 4 is in the shape of a truncated cone, which fits to the inner size of the neck tapered hole section 51. Since there is an interference fit between the neck rod 4 and the neck tapered hole section 51, when the neck rod 4 is completely inserted into the neck tapered hole section 51, the connection between them can be accomplished.

The pin tapered hole section 52 is used for the center lock pin 9 to pass through, and is fixed with the tail part of the center lock pin 9. Preferably, the tail part of the center lock pin 9 is in the shape of a truncated cone, which fits to the inner size of the pin tapered hole section 52. Since there is an interference fit between the tail part of the center lock pin 9 and the pin tapered hole section 52, when the mail part of the center lock pin 9 is inserted into the pin tapered hole section 52 and the tail part of the center lock pin 9 is embedded therein, the connection between them can be accomplished.

The dimension of the contact position between the neck tapered hole section 51 and the pin tapered hole section 52 is abruptly changed, that is, the maximum cross-sectional area of the pin tapered hole section 52 located below is smaller than the minimum cross-sectional area of the neck tapered hole section 51 located above, so that the neck rod 4 cannot be inserted into the pin tapered hole section 52, preventing the neck rod 4 and the center lock pin 9 from interfering with each other.

In a preferred embodiment, the conicity of the neck tapered hole section 51 is in a range from 1:17 to 1:25, preferably 1:22. The inventors found that when the conicity of the neck tapered hole section 51 is 1:22, it can be tightly fixed with the lower end of the neck rod 4 , and the lower end of the neck rod 4 also can be easily inserted into the neck tapered hole section 51, accordingly reducing the installation difficulty.

In a preferred embodiment, the conicity of the pin tapered hole section 52 is in a range from 1:13 to 1:20, preferably 1:15. The inventors found that when the conicity of the pin tapered hole section 52 is 1:15, it can be tightly fixed with the tail part of the center lock pin 9, and the main part of the center lock pin 9 also can be easily inserted into the pin tapered hole section 52, accordingly reducing the installation difficulty.

In a preferred embodiment, as shown in FIG. 4, a through hole is provided inside the cylindrical auxiliary fixed table 62. The through hole is a tapered hole 63, and the tail part of the tail pin 8 in the shape of a truncated cone just can be embedded into the tapered hole 63. After the tail pin is screwed into the femur, the tail part of the tail pin is in contact with the tapered hole 63, so that the tail pin can be fully contacted with the auxiliary fixed table 62, accordingly ensuring the firmly connection between the tail pin and the auxiliary fixed table. The thickness of the auxiliary fixed table 62 is about 2/3 of the thickness of the greater trochanter, which can substantially ensure the firmly connection between the auxiliary fixed table 62 and the tail pin 8 and can also ensure that the auxiliary fixed table 62 does not cause excessive damage to the greater trochanter.

Preferably, the front parts of the center lock pin 9 and the tail pin 8 are both provided with threads, so as to be firmly fixed to the femoral tissue by screwing.

In a preferred embodiment, a through hole is also provided on the wall of the wing-shaped cup body 6. The femoral neck inside the wing-shaped cup body 6 can be exposed via the through hole, which can prevent the nourishing blood vessels on the femoral neck from being subjected to the shear force and damaged to maintain the normal the physiological function.

Some existing stemless hip joint prostheses are also provided with the through hole structures to reduce the damage to the nourishing blood vessels. However, the effects are not as desired due to the various physiological structures and positions of the tissues (such as nourishing blood vessels) of different people.

Therefore, in the present invention, preferably, a large window structure is provided at the region where the nourishing blood vessels are concentrated. That is, the front of the wing-shaped cup body 6 is opened to expose the femoral tissue inside the cup body 6 including ascending carotid artery and its surrounding biologically active soft tissue, which can improve postoperative recovery and reduce complications. Preferably, when the wing-shaped cup body 6 is installed, its edge can be partly removed suitably to enlarge the opening area of the large window, so that the native nourishing blood vessels can be protected from being pressed and destroyed by the edge of the wing-shaped cup body 6 and accordingly the recovery after surgery can be improved.

Existing stemless prostheses have no pins inserted into the medullary cavity, so their application can be not limited by the age of 45 and can be performed to younger patients. However, their service life is still limited, even if they can be used for 30-40 years, but for young patients, there still exists the problems that the service life will be expired after a few decades, and the replacement is difficult and has a huge risk, which are not taken account by the existing stemless and stemmed prostheses, accordingly leading to the unsatisfied recovery effect after surgery.

In a preferred embodiment, the wing-shaped cup body 6 has a larger inner size and an irregularly curved contour, so that the femoral tissues are subjected to less damage during the installation of the wing-shaped cup body 6, only a few of nourishing blood vessels are injured after surgery, a stemmed hip joint prosthesis still can be installed after the hip joint prosthesis described herein is removed when its service life has expired.

In the description of the present invention, it should be noted that the terms "upper," "lower," "inner," "outer," "front", "rear," etc. indicate the orientation or positional relationship based on the working state of the present invention. The orientation or positional relationship is only for the convenience of describing the present invention and simplifying the description, rather than indicating or implying that the device or element referred to must have a specific orientation, be constructed and operated in a specific orientation, and therefore cannot be understood as limiting the present invention.

The present invention has been described above by combing the preferred embodiments, which are exemplary and only function for illustrations. On the basis of the present invention, various replacements and improvements can be made for the present invention, and they all fall within the scope of protection of the present invention.

## Claims

1. A wing-shaped stemless hip joint prosthesis, **characterized in that**,
the prosthesis comprises a wing-shaped cup body (6) that can be tightly covered outside the femoral neck and the intertrochanter (31);
the front of the wing-shaped cup body (6) is opened, accordingly exposing the residual end of the femoral neck therein;
a lesser trochanter protecting wing (10) is extended obliquely downward from the front of the wing-shaped cup body (6) and can be tightly buckled outside the lesser trochanter (32).

2. The wing-shaped stemless hip joint prosthesis according to claim 1,
**characterized in that**,
a cup wing (7) is extended obliquely downward from the side of the wing-shaped cup body (6) and can be fixed on the intertrochanter line (33).

3. The wing-shaped stemless hip joint prosthesis according to claim 1,
**characterized in that**,
a cylindrical main fixed table (5) is provided inside the wing-shaped cup body (6) and is used to fix an artificial spherical end (1) and a center lock pin (9) on the wing-shaped cup body (6).

4. The wing-shaped stemless hip joint prosthesis according to claim 1,
**characterized in that**,
a tail wing (61) is extended from the back of the wing-shaped cup body (6), and a cylindrical auxiliary fixed table (62) is provided below the tail wing;
the joint prosthesis further comprises a tail pin (8), the front of which passes through the wing-shaped cup body (6), and then is screwed into the proximal end of the femoral tissue near the greater trochanter.

5. The wing-shaped stemless hip joint prosthesis according to claim 3,
**characterized in that**,
the top structure of the artificial spherical end (1) is spherical, and the bottom of the artificial spherical end (1) is provided with a through hole;
the joint prosthesis further comprises a neck rod (4), one end of the neck rod is inserted into the through hole of the artificial spherical end, and the other end is inserted into the main fixed table (5) of the wing-shaped cup body (6).

6. The wing-shaped stemless hip joint prosthesis according to claim 5,
**characterized in that**,
a buffer device (2) is provided inside the artificial spherical end (1), and one end of the buffer device (2) is in contact with the inner wall of the artificial spherical end (1), and the other end is in contact with the neck rod (4).

7. wing-shaped stemless hip joint prosthesis according to claim 3,
**characterized in that**,
a through hole is provided inside the main fixed table (5), and includes a neck tapered hole section (51) and a pin tapered hole section (52);
the neck rod (4) is inserted into the neck tapered hole section (51) and fixed thereto;
the center lock pin (9) enters through the pin tapered hole section (52) and the tail part of the center lock pin (9) is fixed to the pin tapered hole section (52).

8. The wing-shaped stemless hip joint prosthesis according to claim 3,
**characterized in that**,
the wall of the wing-shaped cup body (6) is further provided with a through hole, through which the femoral neck inside the wing-shaped cup body (6) can be exposed.

9. The wing-shaped stemless hip joint prosthesis according to claim 1,
**characterized in that**,
the front of the wing-shaped cup body (6) is opened to expose ascending carotid artery and its surrounding biologically active soft tissue.
